# EUROPÄISCHE PATENTSCHRIFT

(11) **EP 1 812 364 B1**
(45) Veröffentlichungstag und Bekanntmachung des Hinweises auf die Patenterteilung: **08.02.2017**
(21) Anmeldenummer: 05806363.7
(22) Anmeldetag: 27.10.2005
(51) Int. Cl.: C07C 7/04, C07C 7/08, C07C 1/20

(54) **VERFAHREN UND VORRICHTUNG ZUR HERSTELLUNG VON NIEDEREN OLEFINEN AUS OXYGENATEN**
METHOD AND DEVICE FOR PRODUCING LOWER OLEFINS FROM OXYGENATES
PROCEDE ET DISPOSITIF POUR PRODUIRE DES OLEFINES INFERIEURES A PARTIR DE COMPOSES D'OXYGENE

(30) Priorität: 02.11.2004 DE 102004052828
(43) Veröffentlichungstag der Anmeldung: 01.08.2007
(73) Patentinhaber: Air Liquide Global E&C Solutions Germany GmbH, 60439 Frankfurt am Main (DE)
(72) Erfinder: BIRKE, Gerhard, 60389 Frankfurt am Main (DE); KOEMPEL, Harald, 63263 Neu-Isenburg (DE); LIEBNER, Waldemar, 61440 Oberursel (DE); BACH, Hermann, 56412 Heiligenroth (DE)
(86) Internationale Anmeldenummer: PCT/EP2005/011530
(87) Internationale Veröffentlichungsnummer: WO 2006/048184

(56) Entgegenhaltungen:
- WO-A-01/92190
- DE-A1- 10 027 159

## Beschreibung

Die Erfindung betrifft ein Verfahren und eine Vorrichtung zur Herstellung von niederen Olefinen aus Oxygenaten nach dem Oberbegriff des Anspruchs 1.

In dem zugrunde liegenden Verfahren wird auf der sogenannten "Gas to Chemicals" - Route (GTC) aus Methanol, das in einem vom Anmelder entwickelten Verfahren "Lurgi MegaMethanol" ® durch .Umwandlung aus Erdgas gewonnen wird, im ebenfalls vom Anmelder entwickelten sogenannten "Methanol to Propylene" - Verfahren (MTP®) das Zielprodukt Propylen gewonnen.

Ein Verfahren der genannten Art ist nach DE 100 27 159 A1 bekannt. Dabei wird Methanoldampf an einem ersten Katalysator zu einem Dimethyläther (DME) enthaltenden Dampfgemisch umgesetzt aus welchem an einem formselektiven Zeolith-Katalysator ein Propylen enthaltendes erstes Produktgemisch erzeugt wird. Mittels eines Trennsystems wird daraus ein bis 99.5 Vol-% Propylen enthaltender Produktstrom abgezogen während die Restprodukte getrennt abgeführt werden oder auch zum Teil wieder in den formselektiven Zeolith-Katalysator zur erneuten Umsetzung zurückgeführt werden können.

Nachteilig ist dem Stand der Technik, dass die Rückführung der Restprodukte zur erneuten Umsetzung in den Zeolith-Katalysator nur uneffektiv gelingt. WO-A- 01/92190 beschreibt ein Verfahren zur Herstellung von Propylen aus Methanol.

Der Erfindung liegt daher die Aufgabe zugrunde, ein Verfahren und eine Einrichtung der eingangs genannten Art so weiter zu entwickeln, dass die Rückführung der Restprodukte bei der Herstellung von niederen Olefinen effektiver betrieben werden kann.

Diese Aufgabe wird durch das in Anspruch 1 bestimmte Verfahren ebenso wie durch die in Anspruch 11 bestimmte Vorrichtung gelöst. Vorteilhafte Ausgestaltungen der Erfindung sind in den Unteransprüchen gegeben.

Erfindungsgemäß ist die Aufgabe, bei einem Verfahren zur Herstellung von niederen Olefinen aus einem Oxigenate enthaltenden ersten Reaktionsgemisch durch Umsetzen an einem Katalysator zu einem Olefine und Paraffine enthaltenden zweiten Reaktionsgemisch, wobei das zweite Reaktionsgemisch durch ein Trennsystem geführt wird, in dem ein die niederen Olefine enthaltender erster Produktstrom entzogen wird und der dazu verbleibende Produktstrom mindestens teilweise dem Katalysator zugeführt wird, dadurch gelöst, dass durch das Trennsystem dem zweiten Reaktionsgemisch mindestens eine paraffinangereicherte Fraktion entzogen wird, die nicht dem Katalysator zugeführt wird.

Niedere Olefine sind vorzugsweise Propen, aber auch Ethen und Buten. Oxygenate sind vorzugsweise Methanol und daraus über einen Dehydratisierungskatalysator hergestellter Dimethyläther. Denkbar sind auch höhere Alkohole, bzw. deren einfache oder gemischte Äther. In untergeordneten Anteilen können eine Reihe von anderen Oxygenaten, u.a. Ketone und Ester enthalten sein, die als Nebenprodukte in den Alkoholen enthalten sind oder am Katalysator entstehen und zurückgeführt werden. Bisher wurde der dem Katalysator zurückgeführte Strom nicht nach Paraffinen und Olefinen unterschieden. Es zeigte sich, dass die Paraffine kaum vom Katalysator umgesetzt werden und sich daher als Inerte im Rückführungskreislauf anreichern. Daher musste stets ein Teil des Umsatzes im Rückführungskreislauf aus diesem entfernt werden, um die Anreicherung der Inerte zu verhindern. Mit der teilweisen Entfernung des Umsatzes im Rückführungskreislauf wurden auch darin enthaltene am Katalysator umsetzbare Anteile aus dem Umlauf entfernt.

Durch die Abtrennung von Paraffinen vor der Rückführung des verbleibenden Produktstromes im Trennsystem gelingt es, den Umlauf von Inerten im Rückführungskreislauf zu minimieren und den Anteil von am Katalysator umsetzbarer Stoffe zu erhöhen. Damit müssen diese nicht wie bisher zusammen mit den Inerten teilweise abgeführt werden, sondern stehen weiter zur Umsetzung in das Zielprodukt zur Verfügung. Somit gelingt eine Steigerung der Menge der herzustellenden niederen Olefine bei gleicher Menge des eingesetzten ersten Reaktionsgemisches.

Die Trenneinrichtung umfasst dabei günstigerweise eine Paraffin-Olefin-Trennstufe, die der Abtrennung des niedere Olefine enthaltenden ersten Produktstromes nachgeschaltet ist. Dies hat den Vorteil, dass für die Paraffinabtrennung mittels der Paraffin-Olefin-Trennstufe bereits eine erhöhte Konzentration an Paraffinen vorliegt.

Das durch Umsetzen an dem Katalysator erhaltene zweite Reaktionsgemisch enthält im allgemeinen auch kleinere Anteile an Oxygenaten, die als Nebenprodukt am Katalysator entstehen. Diese werden jeweils mit dem verbleibenden Produktstrom zusammen mit den KW-Fraktionen mindestens teilweise zum Katalysator zurückgeführt. Dies hat zum einen den Vorteil, dass auch diese Oxigenate am Katalysator zu den gewünschten niederen Olefinen umgebaut werden können und so die Ausbeute erhöhen. Daneben ergibt sich zum anderen der Vorteil, dass die so zur erneuten Umsetzung zurückgeführten Oxigenate nicht die aus dem Kreislauf entzogene paraffinangereicherte Fraktionen verunreinigen, sodass deren weitere Verwertung vereinfacht wird.

Erfindungsgemäß ist des weiteren die oben genannte Aufgabe bei einem zweiten Verfahren zur Herstellung von niederen Olefinen aus einem Oxigenate enthaltenden ersten Reaktionsgemisch durch Umsetzen an einem Katalysator zu einem Olefine und Paraffine enthaltenden zweiten Reaktionsgemisch dadurch gelöst, dass das zweite Reaktionsgemisch durch eine erste Trenneinrichtung geführt wird, in der ein die niederen Olefine enthaltener erster Produktstrom und ein höhere Olefine und Paraffine enthaltender zweiter Produktstrom abgetrennt wird wobei dem zweiten Produktstrom durch eine zweite Trenneinrichtung die höher siedenden Kohlenwasserstoffe entfernt werden und dem dazu verbleibenden dritten Produktstrom durch eine Extraktivdestillation unter Verwendung eines selektiven polaren Lösungsmittels eine paraffinangereicherte Fraktion entzogen wird und wobei der dazu verbleibende vierte Produktstrom mindestens teilweise zum Katalysator zurückgeführt wird.

Durch den so beschriebenen Betrieb entfalten sich die bereits oben genannten Vorteile. Die paraffinangereicherte Fraktion kann dabei von sehr hoher Reinheit eingestellt werden, so dass sie nicht aufwendig aufbereitet werden muss, sondern direkt zur kommerziellen Verwertung abgegeben werden kann.

Die Abtrennung der im Vergleich zum Gesamtgemisch höher siedenden Kohlenwasserstoffe mittels der zweiten Trenneinrichtung hat den Zweck, dass Aromate weitgehend aus dem Rückführungskreislauf entfernt werden. Aromaten verhalten sich am Katalysator hinsichtlich einer Olefinbildung inert. Sie reagieren aber zu substituierten Aromaten, verbrauchen damit die im ersten Reaktionsgemisch enthaltenen Oxygenate und sind deshalb in den Rückführungsströmen unerwünscht. Daneben können die Aromaten den Katalysator durch Aufkohlung schädigen. Auch würden die Aromaten in einer nachfolgenden Extraktivdestillation die Wirkung des selektiven polaren Lösungsmittels okkupieren und damit die Wirkung des Lösungsmittels auf die Olefine verringern. Durch die Abtrennung der Aromaten wird somit auch Lösungsmittel eingespart.

Nach einer Weiterbildung der Erfindung ist vorgesehen, dass mittels der zweiten Trenneinrichtung dem zweiten Produktstrom eine KW-Fraktion mittlerer Siedelage entzogen wird, so dass diese nicht dem Katalysator zugeführt wird.

Dadurch wird ein Teil des für den Rückfluss vorgesehenen Umsatzes entfernt. So wird sichergestellt, dass die Inerte, die nicht zusammen mit der paraffinangereicherten Fraktion bei der Extraktivdestillation entfernt werden können, teilweise wieder aus dem Rückführungskreislauf ausgeschleust werden und sich somit nicht übermäßig in diesem anreichern.

Durch eine geeignete Aufteilung der leichter siedenden Kohlenwasserstofffraktionen im dritten Produktstrom und der KW-Fraktion mittlerer Siedelage in der Trenneinrichtung wird sichergestellt, dass nur der Teil der Kohlenwasserstoffe der Extraktivdestillation zugeführt wird, der getrennt werden soll. Dabei kann die KW-Fraktion mittlerer Siedelage auch teilweise oder ganz dem Katalysator direkt zurückgeführt werden.

Besonders vorteilhaft wird dem vierten Produktstrom durch eine weitere Destillation eine erste olefinangereicherte Fraktion entzogen und dem verbleibenden fünften Produktstrom durch eine zweite Extraktivdestillation unter Verwendung eines selektiven polaren Lösungsmittels eine zweite paraffinangereicherte Fraktion entzogen und der dazu verbleibende sechste Produktstrom sowie die erste olefinangereicherte Fraktion mindestens teilweise dem Katalysator zurückgeführt. Sinnvollerweise wird mittels eines Lösungsmittelstrippers das Lösungsmittel aus dem sechsten Produktstrom vor der Rückführung zum Katalysator entfernt und der Extraktivdestillation wieder zugegeben.

Dabei kann ein Teil der Kohlenwasserstofffraktion mittlerer Siedelage, der durch die zweite Trenneinrichtung aus dem zweiten Produktstrom entfernt wurde, zusammen mit dem fünften Produktstrom der zweiten Extraktivdestillation aufgegeben werden.

Durch den mehrstufigen Trennprozess wird eine weitere paraffinangereicherte Fraktion aus dem Rückführungskreislauf ausgeschleust. Diese kann ebenfalls von so hoher Reinheit sein, dass sie direkt kommerziell verwertet werden oder mit lediglich geringem Aufwand dazu aufbereitet werden kann. Darüber hinaus kann der Anteil des mittels der zweiten Trenneinrichtung aus dem Rückführungskreislauf auszuschleusenden Menge verringert werden. Ebenso kann der Anteil der verschiedenen Fraktionen, die aus dem Kreislauf ausgeschleust werden, an die augenblickliche Ausbeutestruktur des Katalysators angepaßt und damit der Gesamtkresilauf optimiert werden.

Zweckmäßigerweise wird das Lösungsmittel in den Produktströmen nach der ersten und zweiten Extraktivdestillation mit einem gemeinsamen Lösungsmittelstripper nach der zweiten Extraktivdestillation aus dem sechsten Produktstrom extrahiert und vom gemeinsamen Lösungsmittelstripper der ersten und zweiten Extraktivdestillation wieder zugeführt. Die Aufteilung des Lösungsmittels auf die beiden Extraktivdestillationen richtet sich nach Art und der gewünschten Abtrennung der Kohlenwasserstofffraktionen. Es zeigt sich, dass bei der Destillation des vierten Produktstroms die Zusammensetzung der erhaltenen ersten olefinangereicherten Fraktion weitgehend unabhängig von der Anwesenheit von Lösungsmittel ist und dass diese aufgrund der Siedelage frei von Lösungsmittel gewonnen wird. Daher kann von der Entfernung des Lösungsmittels aus dem vierten Produktstromes abgesehen werden und der an sich übliche Lösungsmittelstripper nach der ersten Extraktivdestillation im vierten Produktstrom kann entfallen. Dadurch kann ein gemeinsamer Lösungsmittelstripper nach der zweiten Extraktivdestillation im sechsten Produktstrom verwendet werden. Dies führt zu Kostenvorteilen für die Anlage.

In analoger Weise können durch je eine weitere Extraktivdestillationsstufe und eine nachgeschaltete Destillation eine weitere paraffinangereicherte und eine olefinangereicherte Fraktion mit jeweils höheren Siedelagen gewonnen werden, wenn dies aus wirtschaftlichen Gründen geboten ist. Dabei kann in ebenso analoger Weise ein gemeinsamer Lösungsmittelstripper verwendet werden. Die paraffinangereicherten Fraktionen würden dabei entsprechend aus dem Kreislauf entfernt werden, während die olefinangereicherten Fraktionen jeweils dem Katalysator wieder zurückgeführt werden würden.

Vorteilhafterweise ist das selektive Lösungsmittel N-Methylpyrrolidon (NMP) welches Paraffinen eine höhere Flüchtigkeit als Olefinen gleicher Kohlenstoffatomzahl verleiht.

Daneben sind auch andere selektive polare Lösungsmittel geeignet, in deren Lösung Paraffine eine höhere Flüchtigkeit als Olefine besitzen. So zum Beispiel: Butyrolacton, Nitrile wie Acetonitril, Propionitril, Methoxypropionitril, Ketone wie Aceton, Furfurol, N-alkylsubstituierte niedere aliphatische Säureamide, wie Dimethylformamid (DMF), Diethylformamid, Dimethylacetamid (DMAC), Diethylacetamid, N-Formylmorpholin (NFM), N-alkylsubstituierte cyclische Säureamide (Lactame) wie N-Alkylpyrrolidone im Allgemeinen, insbesondere N-Methylpyrroldon. Im allgemeinen werden N-alkylsubstituierte niedere aliphatische Säureamide oder N-alkylsubstituierte cyclische Säureamide verwendet. Es können jedoch auch Mischungen dieser Lösungsmittel. untereinander, z.B. von N-Methylpyrrolidon mit Acetonitril, Mischungen dieser Lösungsmittel mit Wasser und/oder mit Ethern, z.B. Methyl-tert.-butylether, Ethyl-tert.-butylether, Propyl-tert.-butylether, n- oder iso-Butyl-tert-butylether, oder mit Tetrahydrofuran eingesetzt werden.

Da die Selektivität von NMP bezüglich Olefinen im Vergleich zu der von Paraffinen relativ niedrig ist muss das Verhältnis von Olefinen zu NMP auf etwa 1 zu 10 oder mehr eingestellt werden.

Besonders vorteilhaft ist dabei dem selektiven Lösungsmittel Ethylenglykol (EG) zugebeben, während der zweiten Extraktivdestillation Wasser zugegeben wird. Das Wasser kann dazu sowohl der eigentlichen Extraktivdestillationskolonne als auch dem zugehörigen Lösungsmittelstripper zugegeben werden.

Anstatt Ethylenglykol sind auch andere Glykolmischungen verwendbar. Die Glykolmischung kann auch das selektive polare Lösungsmittel an sich bilden.

Im Katalysator werden in geringem Maße auch niedrig siedende Oxigenate wie zum Beispiel Aceton, Ethanol oder Methylformiat produziert. Diese werden im Rückfluss vom Katalysator ebenso wie höhere Olefine in die gewünschten niederen Olefine umgesetzt. Es ist daher wünschenswert, auch diese Oxygenate wiederum dem Katalysator zuzuführen. Durch die Anwesenheit von EG und Wasser werden die polaren Oxigenate an das Wasser angebunden und dieses an das EG. Hierdurch wird erreicht, dass die Oxygenate weniger flüchtig werden und nicht zusammen mit der niedrigsiedenden paraffinangereicherten Fraktion, sondern mit dem etwas höhersiedendem sechsten Produktstrom abgezogen und so dem Katalysator zugeführt werden können. Reste der Oxigenate im Lösungsmittelkreislauf können mittels der üblichen Lösungsmittelregeneration entfernt werden. Dadurch wird der Rückführungskreislauf insgesamt optimiert.

Günstigerweise wird das oben erwähnte, einer Extraktivdestillation zuzugebende Wasser aus der ersten Trenneinrichtung abgezogen. Dies ist sinnvoll, da in der ersten Trenneinrichtung ohnehin Wasser anfällt und somit eine eigene Wasserversorgung für die Extraktivdestillation unnötig wird.

Vorzugsweise enthalten die Oxigenate im ersten Reaktionsgemisch Dimethyläther und Wasser während der Katalysator ein formselektiver Zeolith-Katalysator vom Pentasiltyp ist wobei die Oxigenate mindestens teilweise mittels eines Dehydratisierungskatalysators aus Methanol erzeugt werden und wobei das gewünschte niedere Olefin Propylen ist.

Erfindungsgemäß ist des weiteren die oben genannte Aufgabe gelöst durch eine Vorrichtung zur Herstellung von mindestens einem niederen Olefin aus einem Oxigenate enthaltenen ersten Reaktionsgemisch mit einem Katalysator zur Umsetzung in ein Olefine und Paraffine enthaltendes zweites Reaktionsgemisch und mit einem mit dem Katalysator in Verbindung stehendem Trennsystem zur Erzeugung mindestens eines mindestens das eine niedere Olefin enthaltenen ersten Produktstroms und eines verbleibenden Produktstroms und mit einer Rückführungseinrichtung zwischen dem Trennsystem und dem Katalysator für den verbleibenden Produktstrom wobei das Trennsystem eine Paraffin-Olefin-Trenneinrichtung umfasst und die damit abgetrennten Paraffine nicht der Rückführungseinrichtung zugeführt werden.

Durch eine solche Vorrichtung entfalten sich die bereits oben genannten Vorteile.

Im Folgenden wird die Erfindung anhand schematischer Darstellungen zu zwei Ausführungsbeispielen näher erläutert. Es zeigen:
- Fig. 1: ein Fließschema des Verfahrens und der Vorrichtung im Prinzip
- Fig. 2: ein Fließschema des Verfahrens und der Vorrichtung mit einer Extraktivdestillation
- Fig. 3: ein Fließschema mit zwei Extraktivdestillationseinheiten

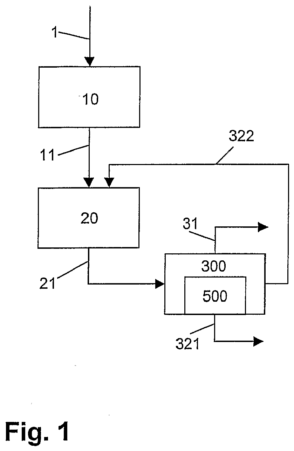

Die Figur 1 zeigt ein Fließschema mit einem DME-Reaktor (10), einem einen Katalysator (20) enthaltenden MTP-Reaktor und einem Trennsystem (300), das eine Olefin-Paraffin-Trenneinrichtung (500) enthält.

Methanol (1) wird als auf etwa 260°C überhitzter Dampf in den DME-Reaktor (10) eingeleitet. Der DME-Reaktor ist ein einstufiger Reaktor in dem der überwiegende Teil des eingeleiteten Methanoldampfs beispielsweise an einem Aluminiumoxidkatalysator (γ-Al₂O₃) nach US 3 058 576 zu einem ersten Reaktionsgemisch (11) aus Dimethylether (DME) und Wasser nach der folgenden Reaktionsgleichung dehydratisiert wird:

2 CH₃OH → CH₃OCH₃ + H₂O

Der genannte Katalysator weist hohe Aktivität und hohe Selektivität auf und erreicht fast thermodynamisches Gleichgewicht. Die Reaktion ist exotherm und das Reaktionsgleichgewicht ist weitgehend unabhängig vom Reaktionsdruck. Durch die Umsetzung im DME-Reaktor (10) wird die Wärmeerzeugung im nachfolgenden MTP-Reaktor (20) abgemildert wodurch sich dessen Effektivität erhöht. Deshalb ist der DME-Reaktor (10) sinnvoll, aber nicht in allen Fällen erforderlich. Neben dem Methanol kann dem DME-Reaktor auch ein Gemisch aus Methanol, DME und Wasser aus der Anlage als Recyclestrom aufgegeben werden.

Das erhaltene erste Reaktionsgemisch (11) wird mit etwa 440°C dem sogenannten MTP-Reaktor zugeführt, der einen Katalysator (20) enthält und das erste Reaktionsgemisch (11) in ein Olefine enthaltendes zweite Reaktionsgemisch (21) umsetzt. Der Katalysator (20) ist beispielsweise ein geschütteter, körniger formselektiver Zeolith-Katalysator vom Pentasiltyp wie aus EP 448 000 B1 bekannt. Dieser besteht aus einer Basis aus Aluminiumsilikaten mit einem Al/Si-Atomverhältnis von mindestens 10, deren Primärkristalliten einen mittleren Durchmesser von 0,1 bis 0,9 µm aufweisen und zu mindestens 20% zu Agglomeraten von 5 bis 500 µm vereinigt sind, wobei die Primärkristallite bzw. Agglomerate durch feinteiliges Aluminiumoxid verbunden sind und wobei die BET-Oberfläche 300 bis 600 m²/g, das Porenvolumen (nach Quecksilberporosimetrie bestimmt) 0,3 bis 0,8 cm²/g und die Menge des Aluminiumoxidbindemittels bezogen auf das Endprodukt 10 bis 40 Gew.-% beträgt und wobei der Katalysator- in H-Form vorliegt. Üblicherweise liegt der Druck im Bereich des Katalysators im Bereich von 1.2 bis 2.0 bar. Günstigerweise liegt die Temperatur der Reaktion am Katalysator bei einer Temperatur von etwa 480°C. Der Reaktor kann aus einer oder mehreren Katalysatorstufen aufgebaut sein wobei die Zuführung zu den einzelnen Katalysatorstufen sowohl jeweils von der vorhergehenden Stufe als auch direkt von der gemeinsamen Zuführung aus erfolgen kann.

Günstigerweise kann dem MTP-Reaktor Prozesswasser aus dem Trennsystem zurückgeführt werden. Dies kann sinnvoll sein, da die zusätzliche Zugabe von Wasser in den MTP-Reaktor den Partialdruck des Einsatzgemisches für die Umsetzung optimieren kann. Dies gilt allgemein auch für die zusätzliche Zugabe von im Bezug auf den Katalysator nicht umsetzbaren Stoffen.

Der Katalysator hat die Eigenschaft, dass er nicht nur die Oxigenate DME oder Methanol in Olefine, sondern auch Olefine in Olefine niedrigerer Kohlenstoffanzahl umwandelt. Die letztgenannte Eigenschaft wird hier ausgenutzt um durch eine qualifizierte Rückführung von Olefinen mit höherer Kohlenstoffanzahl als das hier gewünschte Propylen die Effektivität der Umsetzung des Einsatzes erhöht wird.

Das aus dem MTP-Reaktor erhaltene zweite Reaktionsgemisch (21) enthält üblicherweise Wasser, Olefine, Paraffine, DME, sonstige Kohlenwasserstoffe und sonstige Oxigenate.

Dieses zweite Reaktionsgemisch (21) wird einem Trennsystem (300) zugeführt. Das Trennsystem trennt einen ersten Produktstrom (31) ab, der vor allem aus einem niederen Olefin, hier dem gewünschten Wertprodukt Propylen, besteht. Einen weiteren Teil des eingesetzten zweiten Reaktionsgemisches (21) führt das Trennsystem als verbleibenden Produktstrom (322) zu dem den Katalysator (20) enthaltenden MTP-Reaktor zurück. Dadurch dass dieser Produktstrom Anteile enthält, die am Katalysator in das gewünschte Wertprodukt umgesetzt werden können oder die Reaktion dort positiv beeinflussen, wird durch diese Rückführung die Effektivität des Verfahrens und der Anlage erhöht.

Das Trennsystem weist dabei eine Paraffin-Olefin-Trenneinrichtung (500) auf, die eine paraffinangereicherte Fraktion (321) aus dem Rückführungskreislauf herausführt. Üblicherweise muss bei einem nicht vollständig verarbeitbaren Rückfluss ein Teil dem Rückführungskreislauf entzogen werden, damit sich der Rückführungskreislauf nicht ständig vergrößert. Paraffine sind ein Anteil im zweiten Reaktionsgemisch (21), der am Katalysator nicht mehr umgesetzt werden kann und in nennenswertem Umfang im zweiten Reaktionsgemisch (21) anfallen. Daher erweist sich die selektive Entnahme von vor allem Paraffine als besonders effektiv für den Gesamtprozess.

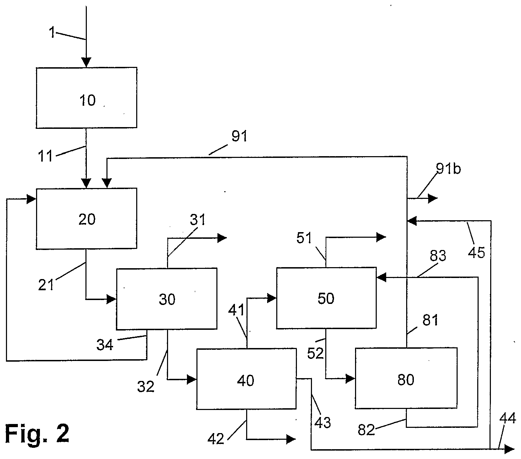

Die Figur 2 zeigt ein Fließschema analog Figur 1, wobei das Trennsystem (300) samt Paraffin-Olefin-Trenneinrichtung näher ausgeführt ist. Dem den Katalysator (20) enthaltenden MTP-Reaktor schließt sich eine erste Trenneinrichtung (30), eine zweite Trenneinrichtung (40) und eine Extraktivdestillation (50) mit einem Lösungsmittelstripper (80) an.

Das zweite Reaktionsgemisch (21) aus dem MTP-Reaktor wird hier einer ersten Trenneinrichtung (30) zugeführt die den Wertstoff Propylen enthaltenden ersten Produktstrom (31) abführt. Daneben trennt die erste. Trenneinrichtung (30) im Reaktionsgemisch enthaltenes Wasser ab (34), welches dem MTP-Reaktor (20) zugeführt wird. Ein Teil des Wassers wird dabei in hier nicht dargestellter Weise aus dem Verfahrenskreislauf entfernt. Der verbleibende zweite Produktstrom (32) wird einer zweiten Trenneinrichtung (40) zugeleitet.

Die erste Trenneinrichtung (30) enthält zur Wasserabtrennung eine Quench-Kolonne und eine Kompressionstufe, der zur weiteren Produktauftrennung eine C3/C4-Trenneinheit nachgeschaltet ist. Über die C3/C4-Trenneinheit wird der erste Produktstrom (31) abgeführt der hier aus einer vor allem olefinhaltigen C3- - Kohlenwasserstofffraktion mit dem Hauptbestandteil Propylen, besteht. Diese kann zur weiteren Aufreinigung einer C2/C3-Trenneinheit und die enthaltene C3-Kohlenwasserstofffraktion einem C3-Splitter aufgegeben werden, mit dem man das gewünschte Propylen abtrennt. Die Trenneinheiten sind üblicherweise Destillationskolonnen.

Aus der C3/C4-Trenneinheit zweigt der zweite Produktstrom (32) ab. Dieser besteht vor allem aus einer C4+ - Kohlenwasserstofffraktion mit erhöhtem Paraffingehalt, sowie aus restlichen Oxygenaten.

Der zweite Produktstrom (32) wird einer zweiten Trenneinrichtung (40) aufgegeben. Diese ist hier als Trennkolonne ausgebildet und trennt eine Fraktion höher siedende Kohlenwasserstoffe (42), vor allem C7+-Kohlenwasserstoffe, und eine Kohlenwasserstofffraktion mittlerer Siedelage (43), vor allem C5-Kohlenwasserstoffe, von einem resultierenden dritten Produktstrom (41) ab.

Die Kohlenwasserstofffraktion mittlerer Siedelage (43) wird über eine Rückführung (45, 91) weitgehend dem Katalysator (20) des MTP-Reaktors wieder zugeführt und über eine Entnahme (44) zu einem geringeren Teil aus dem Kreislauf abgeführt. Durch die direkte Rückführung wird die Mengenbelastung der nachfolgenden Trenneinheiten verringert, was zu ökonomischen Vorteilen führt. Durch die Entnahme (44) wird ein Anreichern von einzelnen Stoffen im Kreislauf verhindert.

Der dritte Produktstrom (41) enthält somit vor allem C4- und C5-Kohlenwasserstoffe, sowohl als Olefine als auch als Paraffine sowie restliche Oxigenate.

Der dritte Produktstrom (41) wird nun einer Extraktivdestillationskolonne (50) zusammen mit einem selektiven Lösungsmittel über einen Lösungsmittelrückfluss (83) zugeführt. Als selektives Lösungsmittel findet hier N-Methylpyrrolidon (NMP) Verwendung. Über Kopf der Extraktivdestillationskolonne (50) wird als eine erste paraffinangereicherte Fraktion (51) etwa 90 bis 95% reine Butan-Isomere abgezogen. Diese werden einer weiteren wirtschaftlichen Verwertung zugeführt. Aus dem Sumpf der Extraktivdestillationskolonne (50) wird ein vierter Produktstrom (52) abgezogen, der vor allem C4-Olefin und C5-Kohlenwasserstoffe sowie das Lösungsmittel enthält. Dieser vierte Produktstrom wird einem Lösungsmittelstripper (80) zugeführt, mit dem das NMP entfernt wird und über einen Lösungsmittelrückfluss wieder der Extraktivdestillationskolonne zurückgeführt wird. Der Rückführung kann in bekannter Weise eine Lösungsmittelaufreinigung zwischengeschaltet sein. Das vom Lösungsmittel befreite Raffinat (81) aus dem Lösungsmittelstripper (80) wird nun zusammen mit dem Rückfluss der Kohlenwasserstofffraktion mittlerer Siedelage (45) dem Katalysator (20) des MTP-Reaktors über einen Rückfluss (91) zurückgeführt. Über eine Entnahme (91 b) kann der Rückführung (91) und damit dem Kreislauf ein Teil des olefinangereicherten Stoffsstroms zwecks Umsetzung der Olefine in einem weiteren Prozeß entzogen werden.

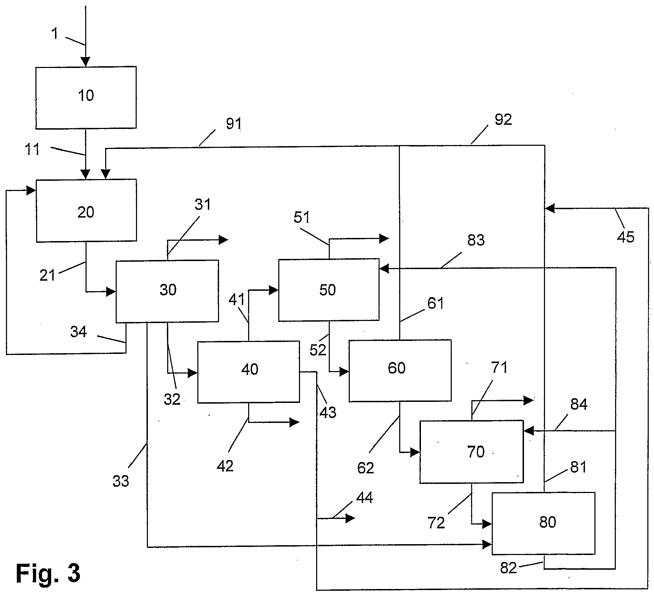

Die Figur 3 zeigt ein Fließschema analog Figur 2, wobei zwischen der Extraktivdestillationskolonne (50) und dem Lösungsmittelstripper (80) zwei weitere Destillationseinrichtungen eingefügt sind, wobei die erste als Destillationskolonne (60) und die nachfolgende als zweite Extraktivdestillation (70) wirkt.

Der vierte Produktstrom (52) wird hier der Destillationskolonne (60) aufgegeben. Über Kopf der Destillationskolonne (60) wird in einer ersten olefinangereicherten Fraktion (61) nahezu der gesamte Anteil an C4-Olefine abgetrennt und dem Katalysator (20) des MTP-Reaktors zurückgeführt. Der über den Sumpf der Destillationskolonne (60) abgezogene fünfte Produktstrom (62) enthält vor allem C5-Kohlenwasserstoffe und wird der zweiten Extraktivdestillationskolonne (70) aufgegeben: Über Kopf der zweiten Extraktivdestillationskolonne (70) wird in einer zweiten paraffinangereicherten Fraktion (71) vor allem ein Teil der C5-Paraffine abgetrennt und aus dem Kreislauf ausgeführt. Der über den Sumpf der zweiten Extraktivdestillationskolonne (70) abgezogene sechste Produktstrom (72) enthält einen Großteil der ursprünglichen C5-Olefine und wird dem Lösungsmittelstripper (80) aufgebeben.

Der Lösungsmittelstripper (80) entfernt über Sumpf das Lösungsmittel aus dem sechsten Produktstrom (72). Das über Kopf erhaltene Raffinat (81) wird über die Rückführung (91, 92) dem Katalysator (20) des MTP-Reaktors zugeführt.

Dem Lösungsmittelstripper (80) wird neben dem sechsten Produktstrom (72) auch noch Wasser in Dampfform aufgegeben, welches über Leitung (33) aus dem Trennsystem (30) abgeführt wird. Alternativ könnte das Wasser auch in flüssiger Form der zweiten Extraktivdestillation (70) zugegeben werden. Dem Lösungsmittelkreislauf ist neben NMP hier Ethylenglykol (EG) zugegeben. Durch die Anwesenheit von EG und Wasser werden die im sechsten Produktstrom (62) enthaltenen polaren Oxigenate wie zum Beispiel Aceton, Ethanol oder Methylformiat über das Wasser als Lösungsvermittler an das EG angebunden. Dadurch wird erreicht, dass die Oxigenate weniger flüchtig werden und in der zweiten Extraktivdestillationskolonne (70) nicht über Kopf mit der zweiten paraffinangereicherten Fraktion (71) abdestilliert und aus dem Kreislauf entfernt werden, sondern im Sumpf verbleiben. Da die Flüchtigkeit der Oxygenate dennoch höher als die von NMP/EG ist, gelangen die besagten Oxigenate dann über Kopf des Lösungsmittelstrippers (80) mit dem Raffinat (81) zurück zum Katalysator (20) des MTP-Reaktors.

Die beiden Ausführungsbeispiele nach den schematischen Darstellungen der Figuren 2 und 3 sind anhand von jeweils einem Beispiel einer Massenbilanz weiter präzisiert

Beispiel 1: Das erste Beispiel zeigt die Abtrennung einer C4-Fraktion bei der Gewinnung von Propylen in einer Konfiguration mit einer Extraktivdestillation nach Figur 2. Die Abtrennung einer C4-Fraktion ist besonders attraktiv, da C4-Kohlenwassserstoffe einen großen Anteil am Reaktionsgemisch (21) haben und zudem C4-Kohlenwasserstoffe dem Siedepunkt nach dem Hauptprodukt Propylen besonders nahe kommen und deren Abtrennung daher die Trennung von C3/C4-Kohlenwassserstoffen merklich erleichtert.

In der Tabelle 1 ist eine Massenbilanz aufgelistet. Die Ströme sind nach Bezugszeichen geordnet. In dieser Konfiguration werden 95.4% der gesamten Olefine (98% der C4-Olefine) abgetrennt und können zum Katalysator zurückgeführt werden. Bei einer konventionellen destillativen Trennung liegt eine vergleichbare Gesamtolefinaubeute bei 85-90%.

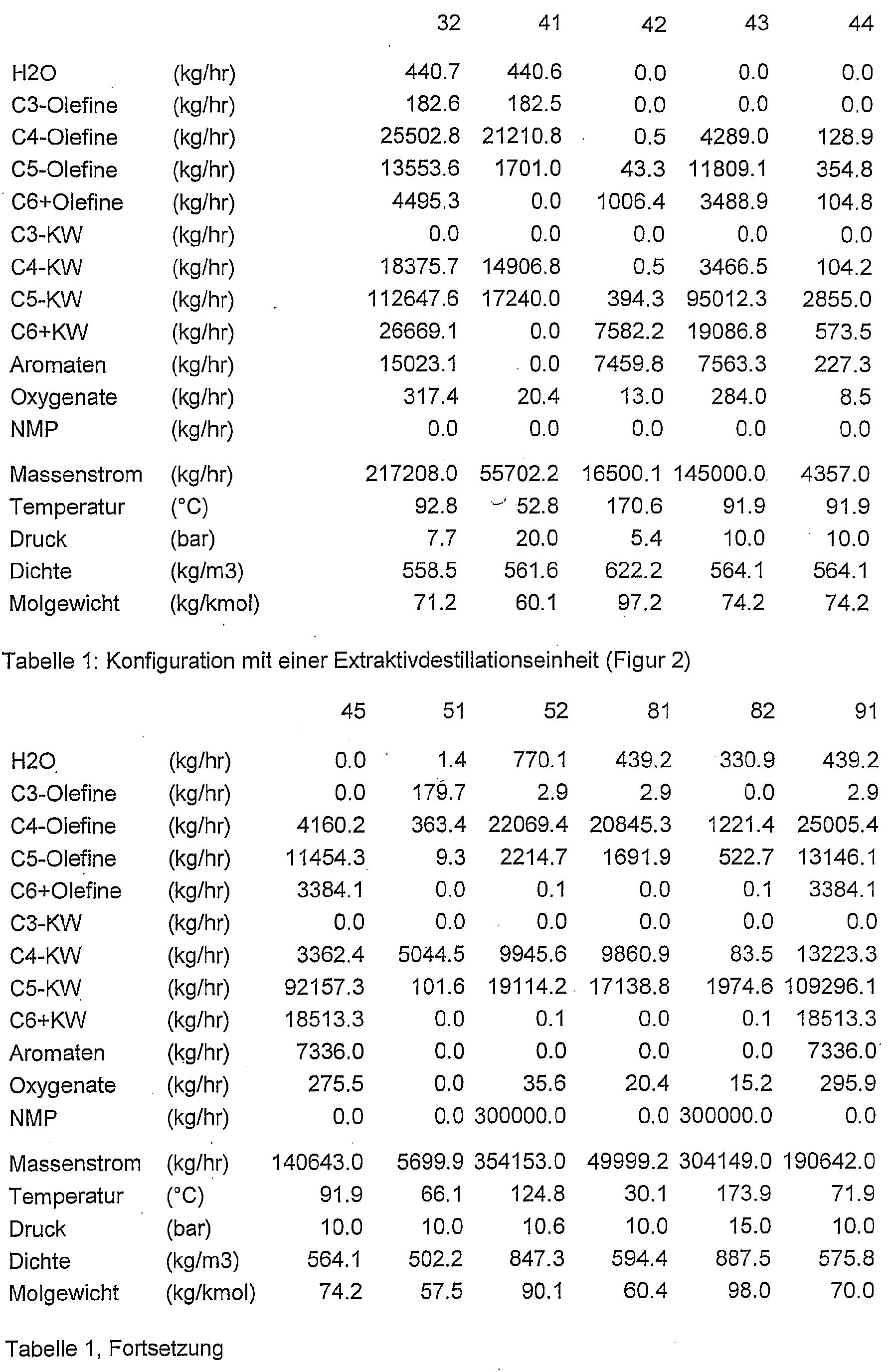

Beispiel 2: Das zweite Beispiel zeigt eine Abtrennung einer C4- und einer C5-Fraktion analog Figur 3. Der Schwerpunkt liegt auch hier bei der C4-Abtrennung, sodass die Hauptmenge des Lösungsmittels hierfür bereitgestellt wird.

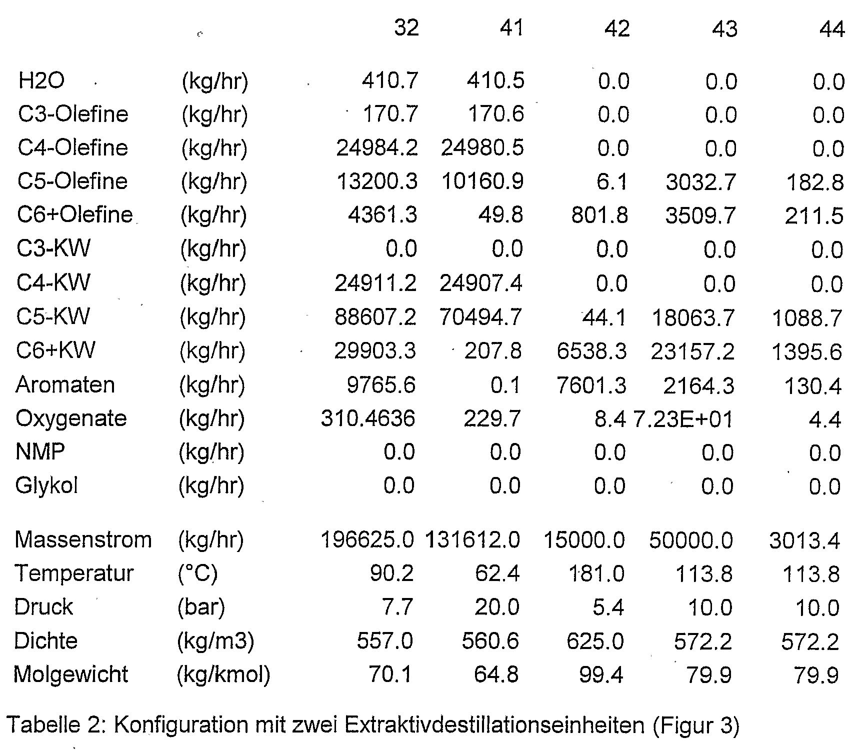

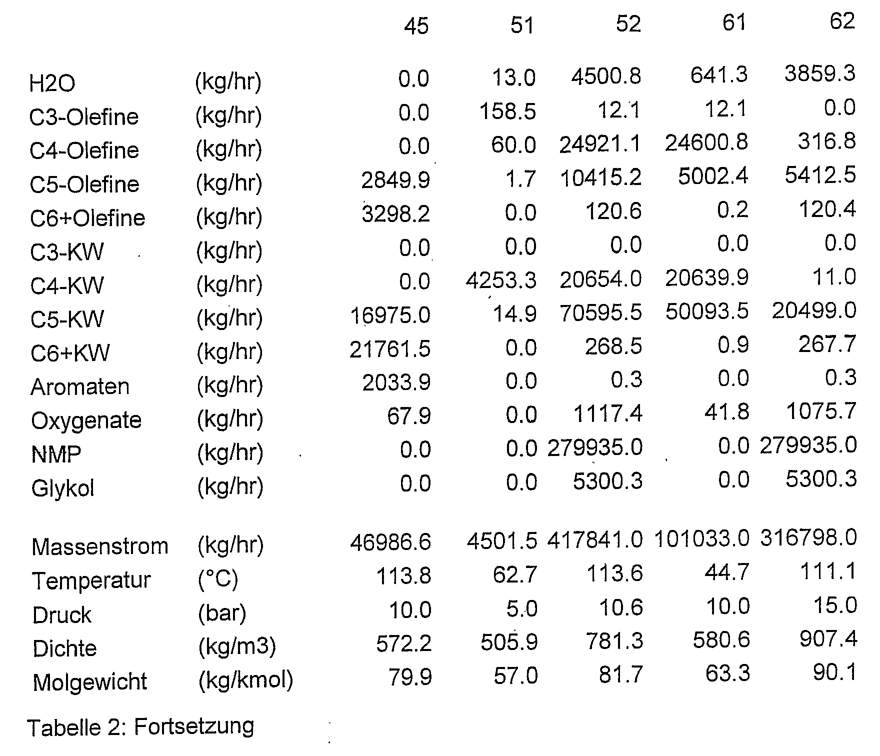

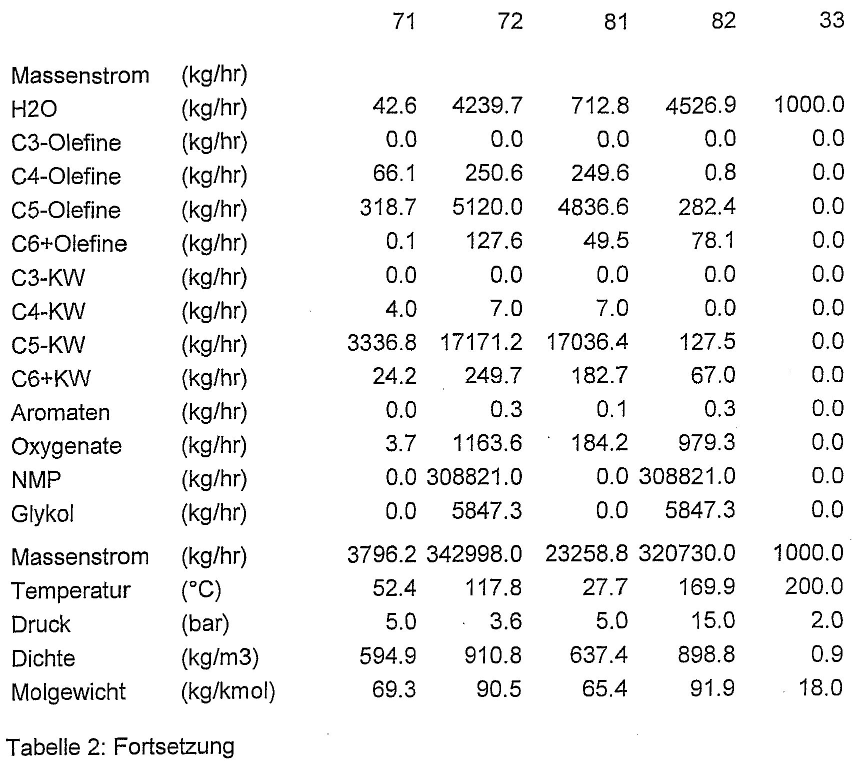

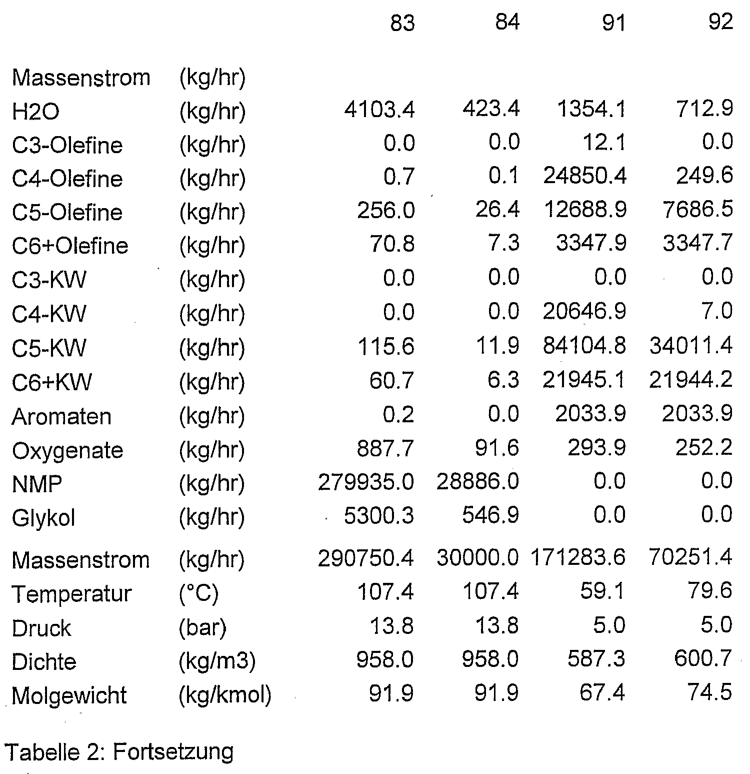

### Liste der Bezugszeichen:

- 1: Methanol
- 10: DME-Reaktor
11 Erstes Reaktionsgemisch
- 20: Katalysator im MTP-Reaktor
21 zweites Reaktionsgemisch
- 300: Trennsystem
321 paraffinangereicherte Fraktion
322 verbleibender Produktstrom
- 30: Erste Trenneinrichtung
31 Erster Produktstrom
32 Zweiter Produktstrom
33 Wasser für Oxygenatentfernung
34 Wasser für Recycle in MTP-Reaktor
- 40: Zweite Trenneinrichtung
41 Dritter Produktstrom
42 Höher siedende KW
43 Kohlenwasserstofffraktion mittlerer Siedelage
44 Entnahme Kohlenwasserstofffraktion mittlerer Siedelage
45 Rückführung Kohlenwasserstofffraktion mittlerer Siedelage
- 500: Paraffin-Olefin-Trenneinrichtung
- 50: Extraktivdestillation
51 Erste paraffinangereicherte Fraktion
52 Vierter Produktstrom
- 60: Destillation
61 Erste olefinangereicherte Fraktion
62 Fünfter Produktstrom
- 70: Zweite Extraktivdestillation
71 Zweite paraffinangereicherte Fraktion
72 Sechster Produktstrom
- 80: Lösungsmittelstripper
81 Raffinat
82 Lösungsmittel
83 Lösungsmittelrückfluss in erste Extraktivdestillation
84 Lösungsmittelrückfluss in zweite Extraktivdestillation
- 91, 92: Rückführung
- 91b: Entnahme

## Patentansprüche

1. Verfahren zur Herstellung von mindestens einem niederen Olefin aus der Gruppe umfassend Ethen, Propen und Buten, aus einem Oxygenate enthaltenden ersten Reaktionsgemisch (11) durch Umsetzen an einem Katalysator (20) zu einem Olefine und Paraffine enthaltenden zweiten Reaktionsgemisch (21), welches durch eine erste Trenneinrichtung (30) geführt wird, in der ein das mindestens eine niedere Olefin enthaltener erster Produktstrom (31) und ein höhere Olefine und Paraffine enthaltender zweiter Produktstrom (32) abgetrennt wird, wobei dem zweiten Produktstrom (32) durch eine zweite Trenneinrichtung (40) die höher siedenden KW (42) entfernt werden und dem dazu verbleibenden dritten Produktstrom (41) durch eine Extraktivdestillation (50, 80) unter Verwendung eines selektiven polaren Lösungsmittels eine paraffinangereicherte Fraktion (51) entzogen wird und wobei der dazu verbleibende vierte Produktstrom (52) mindestens teilweise dem Katalysator (20) zurückgeführt wird (91).

2. Verfahren nach Anspruch 1 wobei dem zweiten Produktstrom (32) durch die zweite Trenneinrichtung (40) eine KW-Fraktion mittlerer Siedelage (43) entzogen wird, so dass diese nicht dem Katalysator (20) zugeführt wird oder mindestens teilweise dem Katalysator (20) zugeführt wird (44).

3. Verfahren nach Anspruch 1 oder 2, wobei dem vierten Produktstrom (52) durch eine weitere Destillation (60) eine erste olefinangereicherte Fraktion (61) entzogen wird und dem verbleibenden fünften Produktstrom (62) durch eine zweite Extraktivdestillation (70, 80) unter Verwendung eines selektiven polaren Lösungsmittels eine zweite paraffinangereicherte Fraktion (71) entzogen wird und wobei der dazu nach Entfernung des Lösungsmittels verbleibende sechste Produktstrom (72) sowie die erste olefinangereicherte Fraktion (61) mindestens teilweise dem Katalysator (20) zurückgeführt wird (91).

4. Verfahren nach Anspruch 3, wobei das Lösungsmittel in den Produktströmen nach der ersten und zweiten Extraktivdestillation (50, 70) mit einem gemeinsamen Lösungsmittelstripper (80) nach der zweiten Extraktivdestillation (70) aus dem sechsten Produktstrom (72) zurückgewonnen und vom gemeinsamen Lösungsmittelstripper der ersten und zweiten Extraktivdestillation wieder zugeführt wird (83, 84).

5. Verfahren nach Anspruch 1 bis 4, wobei das selektive polare Lösungsmittel N-Methylpyrrolidon ist.

6. Verfahren nach Anspruch 3 bis 4, wobei dem selektiven polaren Lösungsmittel Ethylenglykol und Wasser zugegeben wird.

7. Verfahren nach Anspruch 6, wobei das zugegebene Wasser aus der ersten Trenneinrichtung (30) abgezogen wird (33).

8. Verfahren nach einem der vorhergehenden Ansprüche, wobei die Oxygenate im ersten Reaktionsgemisch Dimethylether und Wasser enthalten.

9. Verfahren nach einem der vorhergehenden Ansprüche, wobei der Katalysator (20) für das erste Reaktionsgemisch (11) ein formselektiver Zeolith-Katalysator vom Pentasiltyp ist.

10. Verfahren nach einem der vorhergehenden Ansprüche zur Herstellung von Propylen, wobei das erste Reaktionsgemisch (11) mindestens teilweise mittels eines Dehydratisierungskatalysators (10) aus Methanol (1) erzeugt wird.

11. Vorrichtung zur Herstellung von mindestens einem niederen Olefin aus der Gruppe umfassend Ethen, Propen und Buten, aus einem Oxygenate enthaltenden ersten Reaktionsgemisch (11) mit einem Katalysator (20) zur Umsetzung in ein Olefine und Paraffine enthaltenden zweiten Reaktionsgemisch (21) und mit einem mit dem Katalysator in Verbindung stehendem Trennsystem (300) zur Erzeugung mindestens eines mindestens das eine niedere Olefin enthaltenen ersten Produktstroms und einem verbleibendem Produktstrom (322) und mit einer Rückführungseinrichtung (91) zwischen dem Trennsystem (300) und dem Katalysator (20) für den verbleibenden Produktstrom **dadurch gekennzeichnet, dass** das Trennsystem eine als Paraffin-Olefin-Trenneinrichtung (500) wirkende Extraktivdestillation (50, 80) umfasst, wobei die abgetrennten Paraffine nicht der Rückführungseinrichtung zugeführt werden.

## Claims

1. A method for manufacturing at least one low olefin from the group comprising ethene, propene and butene, from an oxygenate-containing first reaction mixture (11) through conversion at a catalyst (20) to an olefin and paraffin-containing second reaction mixture (21), which is flowed through a first separation stage (30), in which at least one low olefin-containing first product stream (31) and one higher olefin and paraffin-containing second product stream (32) is separated, where from the second product stream (32) the higher boiling hydrocarbons (42) are separated through a second separation stage (40) and from the remaining third product stream (41) a paraffin-enriched fraction (51) is extracted through an extractive distillation (50, 80) by means of a selective polar solvent and where the remaining fourth product stream (52) is recirculated to the catalyst (20) at least partially (91).

2. A method according to claim 1 where from the second product stream (32) a hydrocarbon fraction of middle boiling range (43) is extracted through the second separation stage (40), so that this is not recirculated to the catalyst (20) or is recirculated to the catalyst (20) at least partially (44).

3. A method according to claim 1 or claim 2 where from the fourth product stream (52) a first olefin-enriched fraction (61) is extracted through a further distillation (60) and from the remaining fifth product stream (62) a second paraffin-enriched fraction (71) is extracted through a second extractive distillation (70, 80) by means of a selective polar solvent and where the sixth product stream (72) remaining after the removal of the solvent as well as the first olefin-enriched fraction (61) is recirculated to the catalyst (20) at least partially (91).

4. A method according to claim 3 where the solvent in the product streams after the first and second extractive distillation (50, 70) is reobtained with a common solvent stripper (80) after the second extractive distillation (70) from the sixth product stream (72) and is recirculated to the first and second extractive distillation by the common solvent stripper (83, 84).

5. A method according to claims 1 to 4 where the selective polar solvent is N-Methylpyrrolidone.

6. A method according to claims 3 to 4 where ethylene glycol and water are added to the selective polar solvent.

7. A method according to claim 6 where the added water (33) is extracted from the first separation stage (30).

8. A method according to any one of the preceding claims where the oxygenates in the first reaction mixture contain dimethylether and water.

9. A method according to any one of the preceding claims where the catalyst (20) for the first reaction mixture (11) is a form-selective Zeolith catalyst of the Pentasil type.

10. A method according to any one of the preceding claims for the manufacture of propylene where the first reaction mixture (11) is created from methanol (1) at least partially by means of a dehydrating catalyst (10).

11. Device for manufacturing at least one low olefin from an oxygenates-containing first reaction mixture (11) with a catalyst (20) for conversion to an olefin and paraffin-containing second reaction mixture (21) and with a separation system (300) connected to the catalyst for creation of at least one low olefin-containing first product stream and a remaining product stream (322) and a recycling stage (91) between the separation system (300) and the catalyst (20) for the remaining product stream **characterized in that** the separation system has a extractive distillation (50, 80), working as a paraffin-olefin separation stage (500), where the separated paraffins are not taken to the recycling stage.

## Revendications

1. Procédé de production d'au moins une oléfine inférieure du groupe comprenant l'éthène, le propène et le butène, à partir d'un premier mélange (11) réactionnel contenant des oxygénates par conversion sur un lit catalytique (20) en un deuxième mélange (21) réactionnel, qui contient des oléfines et des paraffines, que l'on fait passer dans un premier dispositif (30) de séparation, dans lequel on sépare un premier courant (31) de produit contenant la au moins une oléfine inférieure et un deuxième courant (32) de produit contenant des oléfines supérieures et des paraffines, on élimine du deuxième courant (32) de produit les hydrocarbures (42) à haut point d'ébullition par un deuxième dispositif (40) de séparation et on retire du troisième courant (41) de produit restant ainsi, par une distillation (50, 80) extractive en utilisant un solvant polaire sélectif, une fraction (51) enrichie en paraffine et on retourne (91) le quatrième courant (52) de produit restant ainsi au lit catalytique (20).

2. Procédé suivant la revendication 1, dans lequel on retire du deuxième courant (32) de produit, par le deuxième dispositif (40) de séparation, une fraction d'hydrocarbures de niveau d'ébullition moyen (43), de sorte que ceux-ci ne sont pas envoyés au lit catalytique (20) ou sont envoyés (44), au moins en partie, au lit catalytique (20).

3. Procédé suivant la revendication 1 ou 2, dans lequel on retire du quatrième courant (52) de produit, par une autre distillation (60), une première fraction (61) enrichie en oléfine et on retire du cinquième courant (62) de produit restant, par une deuxième distillation (70, 80) extractive en utilisant un solvant polaire sélectif, une deuxième fraction (71) enrichie en paraffine et on retourne, au moins en partie, au lit catalytique (20) le sixième courant (72) de produit restant après l'élimination du solvant, ainsi que la première fraction (61) enrichie en oléfine.

4. Procédé suivant la revendication 3, dans lequel on récupère du sixième courant (72) de produit le solvant dans les courants de produit, après la première et la deuxième distillations (50, 70) extractives, par un stripeur (80) de solvant, après la deuxième distillation (70) extractive, et on le renvoie (83, 84) du stripeur commun de solvant à la première et à la deuxième distillations extractives.

5. Procédé suivant les revendications 1 à 4, dans lequel le solvant polaire sélectif et la N-méthylpyrrolidone.

6. Procédé suivant les revendications 3 à 4, dans lequel on ajoute de l'éthylène glycol et de l'eau au solvant polaire sélectif.

7. Procédé suivant la revendication 6, dans lequel on soutire (33) l'eau ajoutée dans le premier dispositif (30) de séparation.

8. Procédé suivant l'une des revendications précédentes, dans lequel les oxygénates du premier mélange réactionnel contiennent de l'oxyde de diméthyle et de l'eau.

9. Procédé suivant l'une des revendications précédentes, dans lequel le lit catalytique (20) pour le premier mélange (11) réactionnel est un lit catalytique au zéolite à sélection de forme du type pentasile.

10. Procédé suivant l'une des revendications précédentes de production de propylène, dans lequel on produit le premier mélange (11) réactionnel, au moins en partie, au moyen d'un catalyseur (10) de déshydratation à partir du méthanol (1).

11. Installation de production d'au moins une oléfine
inférieure du groupe comprenant l'éthène, le propène et le butène, à partir d'un premier mélange (11) réactionnel contenant des oxygénates, comprenant un lit catalytique (20) de conversion en un deuxième mélange (21) réactionnel contenant des oléfines et des paraffines et comprenant un système (300) de séparation en liaison avec le lit catalytique pour produire au moins un premier courant de produit contenant la au moins une oléfine inférieure et comprenant, entre le système (300) de séparation et le lit catalytique (20), un dispositif (91) de recyclage du courant de produit restant, **caractérisée en ce que** le système de séparation comprend une distillation (50, 80) extractive agissant comme dispositif (500) de séparation de paraffines et d'oléfines, les paraffines séparées n'étant pas envoyées au dispositif de recyclage.
